# EUROPÄISCHE PATENTSCHRIFT

(11) **EP 2 007 302 B1**
(45) Veröffentlichungstag und Bekanntmachung des Hinweises auf die Patenterteilung: **14.07.2010**
(21) Anmeldenummer: 06704699.5
(22) Anmeldetag: 16.02.2006
(51) Int. Cl.: A61B 18/16, A61N 1/06

(54) **FLÄCHIGE ELEKTRODE**
PLANAR ELECTRODE
ELECTRODE PLANE

(30) Priorität: 23.02.2005 AT 3062005
(43) Veröffentlichungstag der Anmeldung: 31.12.2008
(60) Teilanmeldung: 10158696.4
(73) Patentinhaber: Nessler Medizintechnik GmbH, 6020 Innsbruck (AT)
(72) Erfinder: NESSLER, Norbert, A-6020 Innsbruck (AT); ERBSE, Stephan, A-6063 Rum (AT)
(74) Vertreter: Dilg, Haeusler, Schindelmann Patentanwaltsgesellschaft mbH
(86) Internationale Anmeldenummer: PCT/AT2006/000057
(87) Internationale Veröffentlichungsnummer: WO 2006/089319

(56) Entgegenhaltungen:
- EP-A- 0 463 196
- WO-A-00/65993
- CA-A1- 1 219 642
- DE-A1- 4 231 236
- US-A- 5 114 424

## Beschreibung

Die Erfindung betrifft eine flächige Elektrode, insbesondere Neutralelektrode, für medizinische Zwecke mit zwei gleich großen, entlang einer linienförmigen Isolierzone elektrisch voneinander getrennten Elektrodenflächen, die entlang einem inneren Randteilbereich unmittelbar aneinandergrenzen.

Unter flächigen Elektroden werden im Sinne der Erfindung alle medizinischen Elektroden zur Anbringung auf der Haut verstanden.

In der WO 00165993 A ist in Fig.5 eine medizinische Neutralelektrode gezeigt, die zwei gleich große halbkreisförmige Elektrodenflächen aufweist, welche entlang einem inneren geradlinigen Randbereich einander gegenüberliegen und durch eine linienförmige Isolierzone voneinander getrennt sind. In der in Fig.8 dieser Druckschrift gezeigten Ausführungsform sind die beiden halbkreisförmigen Elektroden von einer ringartig um diese verlaufenden, bandartigen Elektrode umgeben, die vollkommen elektrisch isoliert von beiden Elektrodenhälften geführt ist. In Fig.3 dieser Druckschrift ist eine Elektrode gezeigt, bei der eine bandförmige Verlängerung einer Elektrodenhälfte sich um beinahe den gesamten restlichen inneren Teil der verschachtelten Elektrodenstruktur erstreckt.

Wie aus der DE 42 31 236 C2 bekannt, kann es während der elektrochirurgischen Behandlung zu einem teilweisen Abheben der Neutralelektrode von der Haut des Patienten und aufgrund der resultierenden stark erhöhten Stromdichte im Gewebe zu schweren Verbrennungen kommen, deren Auswirkungen am Patienten erst nach dem Aufwachen aus der Narkose bemerkt werden.

Um das vollflächige Anliegen der Neutralelektrode sicherstellen zu können, sind Überwachungsvorrichtungen entwickelt worden, die während der Operation oder zwischen den einzelnen Behandlungsschritten Widerstands- oder Symmetriemessungen durchführen.

So verfügen die im Einsatz befindlichen Hochfrequenzgeneratoren üblicherweise über eine Contact Quality Monitoring-(CQM)-Einrichtung, welche unabhängig vom Schneidestrom der chirurgischen Apparatur die korrekte und vollflächige Applikation der Neutralelektrode auf der Haut des Patienten überprüft. Voraussetzung dafür sind zwei- oder mehrteilige Neutralelektroden, die eine Messung der Impedanz zwischen den Elektrodenteilen ermöglichen. Da der Hautwiderstand von Patient zu Patient stark variiert, ist eine eindeutige Aussage, ob die Neutralelektrode richtig aufliegt oder nicht oft nur sehr schwierig und es kann dabei zu Fehlmessungen kommen.

Durch geeignete Maßnahmen, wie z.B. durch eine möglichst lange linienförmige Isolierzone zwischen den Elektrodenteilen, wie dies in der DE 42 31 236 C2 beschrieben ist, läßt sich die Empfindlichkeit der Impedanzüberwachung steigern. Um auch ein distales Abheben der Elektrode erfassen zu können, wird die linienförmige Isolierzone mäandrierend über die gesamte Elektrodenfläche geführt. Eine flächige Ablösung der Elektrode wirkt sich somit annähernd proportional in der von der CQM-Vorrichtung gemessenen Impedanz aus.

Außerdem verfügen einige Hochfrequenzgeneratoren über eine Symmetrieprüfungs-Schaltung, welche über eine Messung des Ableitstromes der Elektrodenteile bestimmt, ob eine symmetrische Stromverteilung vorliegt oder ob ein Elektrodenteil einen höheren Stromanteil führt. Eine solche symmetrische Stromverteilung durch die Neutralelektrode kann mit Hilfe eines geeigneten Designs der Neutralelektrode gefördert werden. Wie sich aber gezeigt hat, reicht es nicht einfach aus, wie bei der Elektrode gemäß DE 42 31 236 C2 die Elektrodenteile mit gleicher Fläche zu dimensionieren, da die Stromaufnahme pro Flächeneinheit der Elektrode stark richtungsabhängig in Bezug auf das Operationsfeld und auch auf die Orientierung des umgebenden Muskelgewebes ist, die dem Fachpersonal bekannt ist.

Aufgabe der Erfindung ist es daher, eine eingangs genannte flächige Elektrode anzugeben, mit der über eine CQM-Proportionalisierung hinaus außerdem auch die Erzielung von symmetrischen Ableitströmen im Anwendungsbetrieb erreicht wird. Erfindungsgemäß wird dies dadurch erzielt, daß die zwei gleich großen Elektrodenflächen jeweils einen bandförmigen Verlängerungsabschnitt aufweisen, welcher die jeweils andere Elektrodenfläche zumindest entlang einem äußeren Randteilbereich umgibt und dabei durch die verlängerte Isolierzone von der jeweils umgebenen Elektrodenfläche getrennt ist, und daß die beiden äußeren Randteilbereiche sich im wesentlichen jeweils ausgehend vom einen Ende des inneren Randteilbereiches um die jeweils andere Elektrodenfläche herum zumindest zum gegenüberliegenden anderen Ende des inneren Randteilbereiches erstrecken. Gemäß der Erfindung sind die zwei gleich großen Elektrodenflächen an ihrem dem inneren Teilstück der linienförmigen Isolierzone gegenüberliegenden Ende halbkreisförmig ausgebildet, welches Ende von den bandförmigen Verlängerungsabschnitten durch die äußeren Teilstücke der linienförmigen Isolierzone jeweils sichelförmig umgeben ist.

Auf diese Weise kann sowohl ein langer Isolierpfad durch die erfindungsgemäße Elektrode erreicht werden, der eine hohe Empfindlichkeit der CQM-Messung ermöglicht als auch eine Elektrodenflächengestaltung gewählt werden, die eine verläßliche Symmetriemessung der Ableitströme während der Operation gewährleistet, da auch in den Randbereichen, die einen überproportionalen Einfluß auf das Meßergebnis haben, die Anteile der Elektrodenfläche gleich groß angeordnet werden können.

Die zwei zentral gelegenen Elektrodenflächen sind dabei von den Verlängerungsabschnitten der jeweils anderen Elektrodenfläche umgeben. Ein relativ langer Isolierpfad und auch eine gegenüber den Randbereichen symmetrische Flächenaufteilung der Elektrodenflächen ist dadurch möglich.

Eine einfache konstruktive Gestaltung ergibt sich, wenn ein inneres Teilstück der linienförmigen Isolierzone und die aneinandergrenzenden inneren Randteilbereiche der zwei gleich großen Elektrodenflächen geradlinig verlaufen und sich entlang einer Nebenachse der flächigen Elektrode erstrecken.

Um die Ausbildung von hohen lokalen Stromdichten während der Anwendung zu verhindern, sind gerundete Elektrodenformen von Vorteil, die kleinere Krümmungsradien in den Randbereichen vermeiden. Für die Anwendung bei Symmetriestrom-Messungen ist eine hohe Flächensymmetrie der Einzeielektroden von Vorteil. In dieser Hinsicht kann ein Merkmal der erfindungsgemäßen Elektrode darin bestehen, daß die zwei Elektrodenflächen um eine Achse senkrecht zur Ebene der flächigen Elektrode zweifach-symmetrisch ausgebildet sind.

Weiters können Anschlußlaschen an einem Ende eines der Verlängerungsabschnitte der Flächenelektroden und einem Übergangsbereich zwischen der anderen der Flächenelektroden und des anderen Verlängerungsabschnittes angeordnet sein. Auf diese Weise kann das Anschließen der erfindungsgemäßen Elektrode an einer Seite erfolgen, wodurch die dieser Seite gegenüberliegende Seite, welche keine Anschlüsse aufweist, dafür geeignet ist, auf dem Patienten in Richtung des Operationsfeldes orientiert zu werden.

Eine vorteilhafte symmetrische Anordnung läßt sich erzielen, wenn die Anschlußlaschen sich an einer Seite der flächigen Elektrode in der gedachten Verlängerung des inneren Teilstückes entlang der Nebenachse erstrecken.

Weiters hat es sich aus mechanischen Gründen und aus Symmetriegründen als günstig erwiesen, wenn die Verlängerungsabschnitte sich jeweils in einem entlang der Nebenachse gelegenen Eckbereich der Flächenelektroden von diesen wegerstrecken, wobei die Eckbereiche gegenüber einer Hauptachse der flächigen Elektrode gleich beabstandet sind, und daß die Verlängerungsabschnitte sich jeweils um die andere der zwei Elektrodenflächen bis im wesentlich in die Nähe des Eckbereiches der anderen Elektrodenfläche erstrecken.

Eine andere Ausführungsform der Erfindung, bei der eine erhöhte Länge der Isolierzone vorliegt, kann darin bestehen, daß sich jeweils entlang der Hauptachse ein Vorsprung der bandförmigen Verlängerungsabschnitte in die jeweils andere Elektrodenfläche erstreckt.

Im zentralen Bereich der erfindungsgemäßen Elektrode ist die Stromdichte während der Anwendung relativ gering, sodaß diese Zone dafür genutzt werden kann, Anschlüsse für die Elektrodenflächen vorzusehen. Dies kann dadurch erreicht werden, daß Anschlußlaschen im mittleren Bereich vorgesehen sind, die mit den Elektrodenflächen verbunden sind.

Diese Anbringung von Anschlußlaschen im zentralen Bereich einer flächigen Elektrode, insbesondere einer Neutralelektrode kann auch für alle bekannten Ausführungsformen dieses Elektrodentyps angewandt werden und wird als gesonderte Erfindung beansprucht.

Eine Vervielfachung des erfindungsgemäßen Prinzips, die innerhalb des Schutzumfangs der Erfindung liegt, kann beispielsweise dadurch erzielt werden, daß die um die jeweils andere Elektrodenfläche geführten Verlängerungsabschnitte anschließend in den Bereich der zugehörigen Elektrodenfläche zurückgeführt sind und dort, getrennt durch die linienförmige Isolierzone, die jeweils anderen Verlängerungsabschnitte umgeben.

Diesbezüglich sind weitere andere Formen der Führung der Verlängerungsabschnitte im Rahmen der Erfindung ausführbar. So kann vorgesehen sein, daß die bandförmigen Verlängerungsabschnitte um die beiden Elektrodenflächen spiralförmig ineinanderverlaufend zwei oder mehrfach herumgeführt sind.

Bei mittiger Anordnung der Anschlußlaschen, wenn diese also direkt mit den beiden zentral angeordneten Elektroden verbunden sind, kann es vorteilhaft sein, wenn der Stromanteil des inneren Bereiches gegenüber jenem des äußeren so beeinflußt wird, daß die sonst vorhandene Stromkonzentration in den Randbereichen der Elektrode sich gleichmäßiger über die gesamte Elektrodenfläche verteilt. Dies kann z.B. durch Vorsehen eines von außen nach innen gerichteten Impedanzgradienten erreicht werden. d.h. daß je weiter der Strom in den Außenbereich der Elektrode fließt sich die Impedanz für ihn erhöht.

Gemäß einer Ausführungsform der Erfindung können zu diesem Zweck entlang des Verlaufes der bandförmigen Verlängerungsabschnitte diese an einer oder mehreren Stellen unterbrochen und Impedanzen zur Ausbildung eines Impedanzgradienten zwischengeschaltet sein.

Nachstehend wird die Erfindung anhand der in den Zeichnungen gezeigten Ausführungsbeispiele eingehend erläutert. Es zeigt dabei
Fig.1 eine schematische Draufsicht auf eine Ausführungsform der erfindungsgemäßen Elektrode;
Fig.2 eine schematische Draufsicht auf eine weitere Ausführungsform der erfindungsgemäßen Elektrode;
Fig.3 eine schematische Draufsicht auf eine weitere Ausführungsform der erfindungsgemäßen Elektrode;
Fig.4 eine schematische Draufsicht auf eine weitere Ausführungsform der erfindungsgemäßen Elektrode;
Fig.5 eine schematische Draufsicht auf eine weitere Ausführungsform der erfindungsgemäßen Elektrode und
Fig.6 eine schematische Draufsicht auf eine weitere Ausführungsform der erfindungsgemäßen Elektrode.

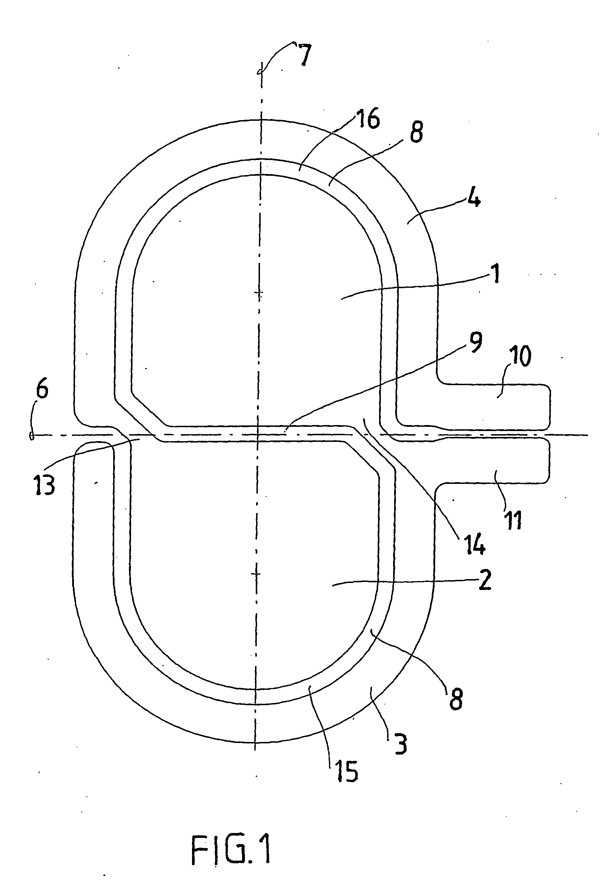

Fig.1 zeigt eine flächige Elektrode, insbesondere eine Neutralelektrode für die Hochfrequenz-Chirurgie. Die beiden durch ausgezogene Linien umrandeten Flächen sind zwei Elektrodenflächen 1, 2, die entlang einer linienförmigen Isolierzone 8 elektrisch voneinander getrennt sind.

Die in Form eines Ausführungsbeispiels gezeigte flächige Elektrode kann in jedem der bekannten Materialien zur Herstellung von Neutralelektroden für den einfachen oder mehrfachen Gebrauch umgesetzt werden. Insbesondere kann die leitfähige Fläche aus einer mit leitfähigem Gel oder Kleber beschichteten Metallfolie bestehen. Die linienförmige Isolierzone 8 ist z.B. 2 bis 5 mm breit und kann entweder völlig frei von leitfähigem Material, oder auch mit schwach querleitfähigem Gel oder Kleber überdeckt sein. Es können im Rahmen der Erfindung auch von diesen Angaben abweichende Bauformen als flächige Elektrode angewandt werden.

Erfindungsgemäß sind die zwei Elektrodenflächen 1, 2 durch die linienförmige Isolierzone 8 voneinander getrennt, zumindest teilweise von bandförmigen Verlängerungsabschnitten 3, 4 der jeweils anderen Elektrodenfläche umschlossen.

Gegenüber den bandförmigen Verlängerungsabschnitten 3, 4, die außenliegend angeordnet sind, nehmen die zwei Elektrodenflächen 1, 2 einen zentralen inneren Bereich der erfindungsgemäßen Elektrode ein.

Die bandförmigen Verlängerungsabschnitte 3, 4 sind im Vergleich zu den zentral angeordneten Elektrodenflächen 1, 2 schmal ausgebildet, d.h. ihre Länge ist deutlich größer als ihre Breite.

Im inneren Bereich grenzen die zwei Elektrodenflächen 1, 2 an einer Seite entlang einem inneren Teilstück 9 der linienförmigen Isolierzone 8 aneinander, und die zwei Elektrodenflächen 1, 2 weisen jeweils einen bandförmigen Verlängerungsabschnitt 3, 4 auf, der sich jeweils um die andere Elektrodenfläche erstreckt.

Die bandförmigen Verlängerungsabschnitte 3, 4 der zwei Elektrodenflächen 1, 2 sind entlang der vom inneren Teilstück 9 ausgehenden äußeren Teilstücke 15, 16 der linienförmigen Isolierzone 8 um die jeweils andere Elektrodenfläche geführt.

Für weitere geometrische Überlegungen ist in Fig.1 eine Hauptachse 7 und eine Nebenachse 6 der erfindungsgemäßen Elektrode eingezeichnet.

Das innere Teilstück der linienförmigen Isolierzone 8 verläuft geradlinig und erstreckt sich entlang einer Nebenachse 6 der flächigen Elektrode.

An ihrem dem inneren Teilstück 9 gegenüberliegenden Ende sind die Elektrodenflächen 1, 2 halbkreisförmig ausgebildet. Diese Enden sind von den Verlängerungsabschnitten 3, 4 durch die äußeren Teilstücke 15, 16 der linienförmigen Isolierzone 8 jeweils sichelförmig umgeben.

In dem in Fig.1 gezeigten Ausführungsbeispiel weist die erfindungsgemäße Elektrode eine ovale Form auf. Auf diese ist die Erfindung jedoch nicht beschränkt, sie kann auch in einer anderen geometrischen Form ausgeführt sein. Sie kann etwa gegenüber der in Fig.1 dargestellten Bauweise beliebig gestaucht oder gedehnt ausgeführt, also von quergeteilt über rund bis längsgeteilt aussehen. Außerdem kann der Abstand der linienförmigen Isolierzone 8 vom Rand in weiten Grenzen variiert werden.

Im wesentlichen sind die zwei Elektrodenflächen 1, 2 um eine Achse senkrecht zur Ebene der erfindungsgemäßen Elektrode, die durch den Schnittpunkt von Haupt- und Nebenachse 7, 6 verläuft zweifach-symmetrisch ausgebildet. Die Art der Symmetrie kann auch anders gestaltet sein.

Zur Abführung des Ableitstromes bzw. zur Einprägung eines Meßstromes sind Anschlußlaschen 10, 11 an einem Ende eines der Verlängerungsabschnitte 3, 4 der Flächenelektroden 1, 2 und einem Übergangsbereich zwischen der anderen der Flächenelektroden 1, 2 und des anderen Verlängerungsabschnittes 3, 4 angeordnet. Die Anschlußlaschen 10, 11 erstrecken sich dabei an einer Seite der flächigen Elektrode in der gedachten Verlängerung des inneren Teilstückes 9 entlang der Nebenachse 6.

Wie weiters in Fig. 1 gezeigt erstrecken sich die Verlängerungsabschnitte 3, 4 jeweils in einem entlang der Nebenachse 6 gelegenen Eckbereich 13, 14 der Flächenelektroden 1, 2 von diesen weg, wobei die Eckbereiche 13, 14 gegenüber der Hauptachse 7 der flächigen Elektrode gleich beabstandet sind. Die bandförmigen Verlängerungsabschnitte 3, 4 verlaufen jeweils um die andere der zwei Elektrodenflächen 1, 2 bis im wesentlich in die Nähe des Eckbereiches 13, 14 dieser anderen Elektrodenfläche.

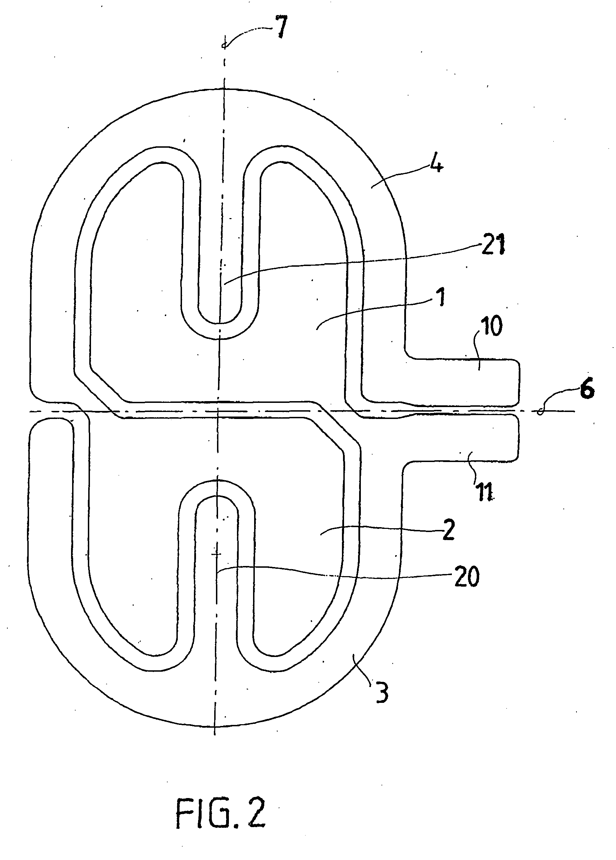

Bei der in Fig.2 gezeigten Ausführungsform erstreckt sich jeweils entlang der Hauptachse 7 ein Vorsprung 20, 21 der bandförmigen Verlängerungsabschnitte 3, 4 in die jeweils andere Elektrodenfläche 1, 2 und verlängert dadurch die linienförmige Isolierzone 8.

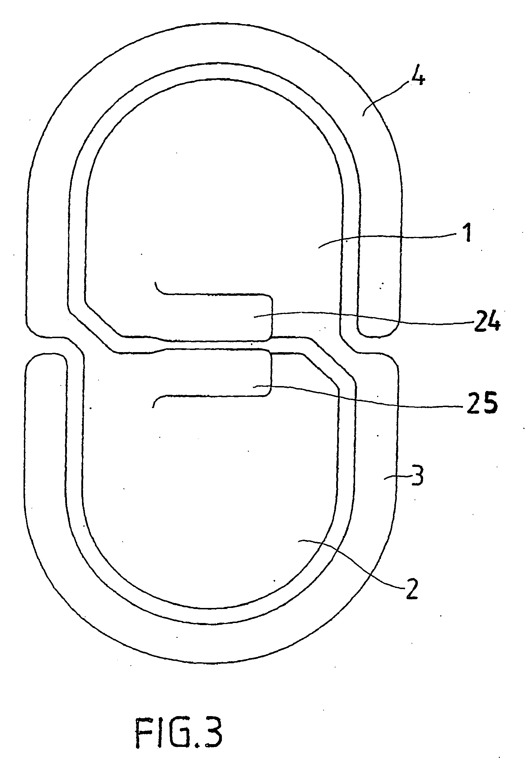

Der mittlere Bereich der erfindungsgemäßen Elektrode nimmt aufgrund des Randeffektes einen vergleichsweise geringen Stromanteil auf und weist daher einen geringeren Wirkungsgrad auf als die Randbereiche. In der in Fig.3 gezeigten Ausführungsform sind daher platz- und materialsparend Anschlußlaschen 24, 25 in diesem mittleren Bereich vorgesehen, welche mit den Elektrodenflächen 1, 2 verbunden sind.

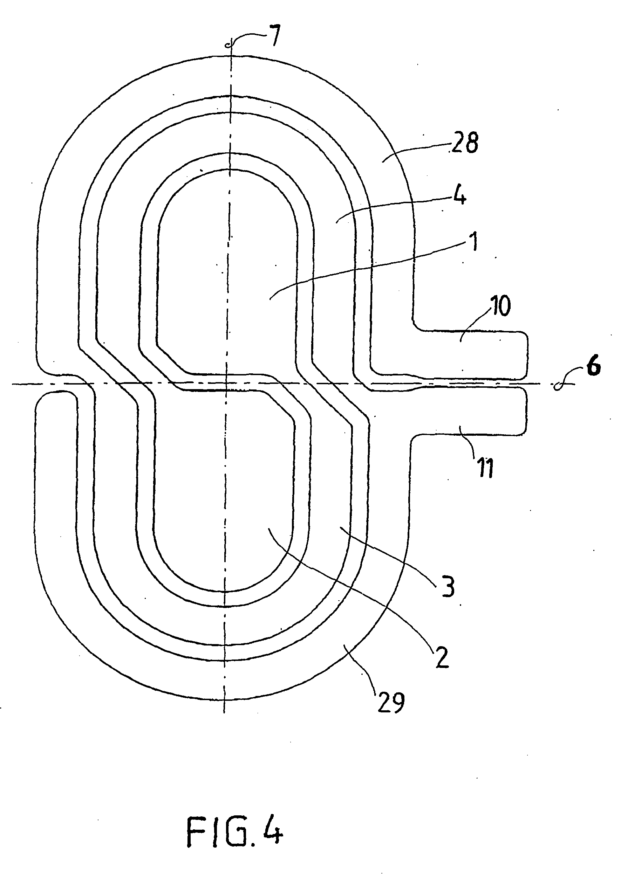

Schließlich sind in der in Fig.4 angegebenen Ausführungsform der erfindungsgemäßen Elektrode die um die jeweils andere Elektrodenfläche 1, 2 geführten Verlängerungsabschnitte 3, 4 anschließend in den Bereich der zugehörigen Elektrodenfläche zurückgeführt und umgeben die jeweils anderen Verlängerungsabschnitte 3,4 in Form von weiter außen liegender Abschnitte 28, 29.

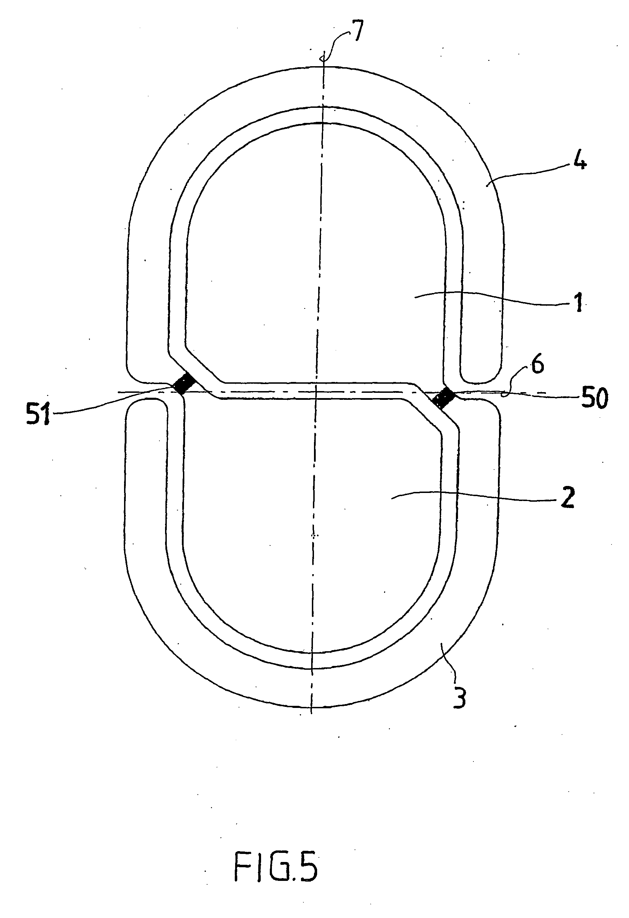

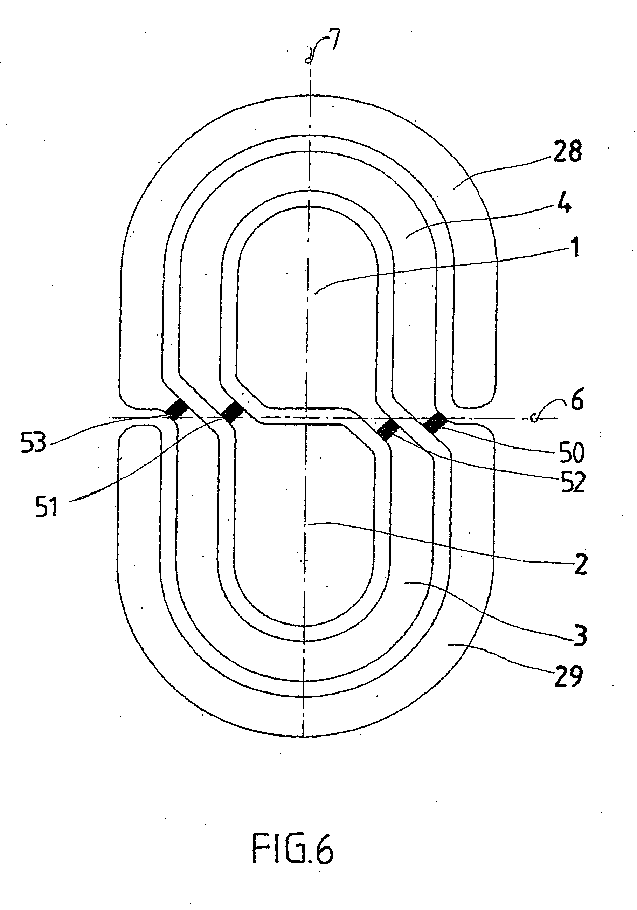

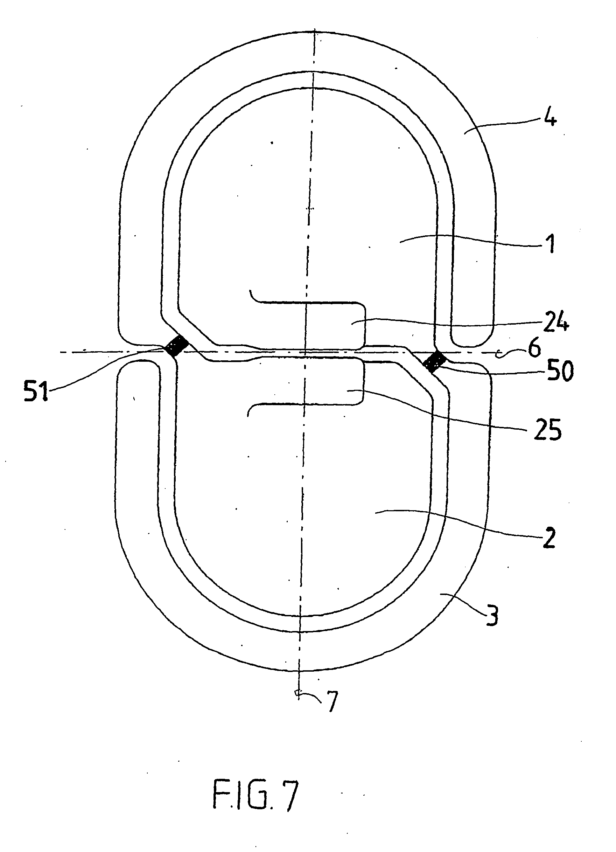

Fig.5, 6 und 7 zeigen weitere Ausführungsbeispiele der Erfindung, bei denen die Anschlußlaschen 24, 25 (Fig.7) mittig angeordnet sind. In den Fig.5 und 6 ist die Anbringung der Anschlußlaschen nicht gezeigt, weil sie auch auf eine andere Art als der in Fig. 7 gezeigten geschehen kann, dies aber nichts zur Erfindung beiträgt.

In den Elektroden gemäß Fig. 5, 6 und 7 sind entlang des Verlaufes der bandförmigen Verlängerungsabschnitte 3, 4 diese an einer oder mehreren Stellen unterbrochen und Impedanzen 50, 51, 52, 53 zur Ausbildung eines Impedanzgradienten zwischengeschaltet. Die Anordndung der Impedanzen kann z.B. so geschehen, daß die Nebenachse 6 durch die Anbringungsstellen der Impedanzen 50, 51, 52, 52 verläuft, wodurch eine schrittweise Erhöhung der Impedanz nach außen hin verwirklicht werden kann. Dadurch läßt sich die Stromverteilung innerhalb der erfindungsgemäßen Elektrode beeinflussen. Die Art, die Dimensionierung und die Anbringungsstellen der Impedanzen 50, 51, 52, 53 kann je nach Bedarf variiert werden.

Die Impedanzen 50, 51, 52, 53 selbst können sowie die Elektrodenflächen 1, 2 und die Verlängerungsabschnitte 3, 4 bzw. die außenliegenden Verlängerungsabschnitte 28, 29 durch Aufdrucken von mehr oder weniger leitfähigen Lacken aufgedruckt werden.

## Patentansprüche

1. Flächige Elektrode, insbesondere Neutralelektrode, für medizinische Zwecke mit zwei gleich großen, entlang einer linienförmigen Isolierzone (8) elektrisch voneinander getrennten Elektrodenflächen (1, 2), die entlang einem inneren Randteilbereich (9) unmittelbar aneinandergrenzen,
wobei die zwei gleich großen Elektrodenflächen (1, 2) jeweils einen bandförmigen Verlängerungsabschnitt (3, 4) aufweisen, welcher die jeweils andere Elektrodenfläche (1, 2) zumindest entlang einem äußeren Randteilbereich (15, 16) umgibt und dabei durch die verlängerte Isolierzone (8) von der jeweils umgebenen Elektrodenfläche (1, 2) getrennt ist,
wobei die beiden äußeren Randteilbereiche (15, 16) sich im wesentlichen jeweils ausgehend vom einen Ende des inneren Randteilbereiches (9) um die jeweils andere Elektrodenfläche (1, 2) herum zumindest zum gegenüberliegenden anderen Ende des inneren Randteilbereiches (9) erstrecken, und
wobei die zwei gleich großen Elektrodenflächen (1, 2) an ihrem dem inneren Teilstück (9) der linienförmigen Isolierzone (8) gegenüberliegenden Ende halbkreisförmig ausgebildet sind, welches Ende von den bandförmigen Verlängerungsabschnitten (3, 4) durch die äußeren Teilstücke (15, 16) der linienförmigen Isolierzone (8) jeweils sichelförmig umgeben ist.

2. Flächige Elektrode nach Anspruch 1, **dadurch gekennzeichnet, dass** ein inneres Teilstück (9) der linienförmigen Isolierzone (8) und die aneinandergrenzenden inneren Randteilbereiche der zwei gleich großen Elektrodenflächen (1, 2) geradlinig verlaufen und sich entlang einer Nebenachse (6) der flächigen Elektrode erstrecken.

3. Flächige Elektrode nach einem der Ansprüche 1 oder 2, **dadurch gekennzeichnet, dass** die zwei Elektrodenflächen (1, 2) um eine Achse senkrecht zur Ebene der flächigen Elektrode zweifach-symmetrisch ausgebildet sind.

4. Flächige Elektrode nach einem der vorhergehenden Ansprüche 1 bis 3, **dadurch gekennzeichnet, dass** Anschlusslaschen (10,11) an einem Ende eines der Verlängerungsabschnitte (3, 4) der Flächenelektroden (1, 2) und einem Übergangsbereich zwischen der anderen der Flächenelektroden (1, 2) und des anderen Verlängerungsabschnittes (3, 4) angeordnet sind.

5. Flächige Elektrode nach Anspruch 4, **dadurch gekennzeichnet, dass** die Anschlusslaschen (10, 11) sich an einer Seite der flächigen Elektrode in der gedachten Verlängerung des inneren Teilstückes (9) entlang der Nebenachse (6) erstrecken.

6. Flächige Elektrode nach einem der vorhergehenden Ansprüche, **dadurch gekennzeichnet, dass** die Verlängerungsabschnitte (3, 4) sich jeweils in einem entlang der Nebenachse (6) gelegenen Eckbereich (13, 14) der Flächenelektroden (1, 2) von diesen wegerstrecken, wobei die Eckbereiche (13, 14) gegenüber einer Hauptachse (7) der flächigen Elektrode gleich beabstandet sind, und dass die Verlängerungsabschnitte (3, 4) sich jeweils um die andere der zwei Elektrodenflächen (1, 2) bis im wesentlich in die Nähe des Eckbereiches (13, 14) der anderen Elektrodenfläche erstrecken.

7. Flächige Elektrode nach einem der vorhergehenden Ansprüche, **dadurch gekennzeichnet, dass** sich jeweils entlang der Hauptachse (7) ein Vorsprung (20, 21) der bandförmigen Verlängerungsabschnitte (3, 4) in die jeweils andere Elektrodenfläche (1, 2) erstreckt.

8. Flächige Elektrode, insbesondere nach einem der Ansprüche 1 bis 3, **dadurch gekennzeichnet, dass** Anschlusslaschen (24, 25) im mittleren Bereich vorgesehen sind, die mit den Elektrodenflächen (1, 2) verbunden sind.

9. Flächige Elektrode nach Anspruch 1, **dadurch gekennzeichnet, dass** die um die jeweils andere Elektrodenfläche (1, 2) geführten Verlängerungsabschnitte (3, 4) anschließend in den Bereich der zugehörigen Elektrodenfläche (1, 2) zurückgeführt sind und dort, getrennt durch die linienförmige Isolierzone (8), die jeweils anderen Verlängerungsabschnitte (3, 4) umgeben.

10. Flächige Elektrode nach Anspruch 1, **dadurch gekennzeichnet, dass** die bandförmigen Verlängerungsabschnitte (3, 4) um die beiden Elektrodenflächen (1, 2) spiralförmig ineinanderverlaufend zwei oder mehrfach herumgeführt sind.

11. Flächige Elektrode nach einem der vorhergehenden Ansprüche, **dadurch gekennzeichnet, dass** entlang des Verlaufes der bandförmigen Verlängerungsabschnitte (3, 4) diese an einer oder mehreren Stellen unterbrochen und Impedanzen (50, 51, 52, 53) zur Ausbildung eines Impedanzgradienten zwischengeschaltet sind.

12. Flächige Elektrode nach Anspruch 11, **dadurch gekennzeichnet, dass** die Nebenachse (6) durch die Anbringungsstellen der Impedanzen (50, 51, 52, 53) verläuft.

## Claims

1. A planar electrode, in particular a neutral electrode, for medical purposes comprising two equal electrode surfaces (1, 2), electrically separated along a linear insulation zone (8), which adjoins directly along an inner edge portion,
wherein said two equal electrode surfaces (1, 2) comprise, respectively, a band-shaped extension section (3, 4), which encloses said other electrode surface (1, 2) at least along an outer edge portion (15, 16) and thereby is separated by said extended insulation zone (8) from said respectively enclosed electrode surface (1, 2),
wherein said two outer edge portions (15, 16) extend substantially, respectively, going out from one end of said inner edge portion (9) around the other electrode surface (1, 2) at least to the opposite other end of said inner edge portion (9),
wherein said two equal electrode surfaces (1, 2) at their end opposite to said inner part (9) of said linear insulation zone (8) are formed semicircular, which end of said band-shaped extension sections (3, 4) is enclosed by the outer parts (15, 16) of said linear insulation zone (8), respectively, in sickle-shaped manner.

2. The planar electrode according to claim 1,
**characterized in that**
an inner part (9) of said linear insulation zone (8) and said adjoining inner edge portions of said two equal electrode surfaces (1, 2) run straight and extend along a minor axis (6) of said planar electrode.

3. The planar electrode according to claim 1 or 2,
**characterized in that**
said two electrode surfaces (1, 2) are formed double symmetric around an axis perpendicular to the plane of said planar electrode.

4. The planar electrode according to one of the preceding claims 1 to 3,
**characterized in that**
terminal lugs (10, 11) are arranged at one end of one of said extension sections (3, 4) of said planar electrodes (1, 2) and a transition region between the other one of said planar electrodes (1, 2) and of said other extension section (3, 4).

5. The planar electrode according to claim 4,
**characterized in that**
said terminal lugs (10, 11) extend at one side of said planar electrode in the imaginary extension of said inner part (9) along said minor axis (6).

6. The planar electrode according to one of the preceding claims,
**characterized in that**
said extension sections (3, 4), respectively, in a corner region (13, 14) situated along said minor axis (6) of said planar electrodes (1, 2) extend away from these, wherein the corner regions (13, 14) opposite a main axis (7) of said planar electrode are equally spaced, and that said extension sections (3, 4) extend, respectively, around the other one of said two electrode surfaces (1, 2) until substantially in the vicinity of the corner region (13, 14) of the other electrode surface.

7. The planar electrode according to one of the preceding claims,
**characterized in that**
a protrusion (20, 21) of said band-shaped extension sections (3, 4) extends, respectively, along said main axis (7) in the respective other electrode surface (1, 2).

8. The planar electrode, in particular according to one of the claims 1 to 3,
**characterized in that**
terminal lugs (24, 25) are provided in the middle region, which are connected with said electrode surfaces (1, 2).

9. The planar electrode according to claim 1,
**characterized in that**
said extension sections (3, 4) guided around said respective other electrode surface (1, 2) subsequently are guided back in the region of the associated electrode surface (1, 2) and there, separated by said linear insulation zone (8), enclose the respective other extension sections (3, 4).

10. The planar electrode according to claim 1,
**characterized in that**
said band-shaped extension sections (3, 4) are guided around said two electrode surfaces (1, 2) intertwining in spiral form two or several times.

11. The planar electrode according to one of the preceding clams,
**characterized in that**
along the course of said band-shaped extension sections (3, 4) these are interrupted at one or at a plurality of positions and impedances (50, 51, 52, 53) are interposed for forming an impedance gradient.

12. The planar electrode according to claim 11,
**characterized in that**
said minor axis (6) extends through said attachment positions of said impedances (50, 51, 52, 53).

## Revendications

1. Electrode plane, en particulier, électrode neutre, à des fins médicales, comportant deux surfaces d'électrodes (1, 2) électriquement séparées l'une de l'autre, de même dimension, le long d'une zone isolante (8) en forme de lignes, qui sont immédiatement adjacentes le long d'une zone de bordure partielle interne (9),
les deux surfaces d'électrodes de même taille (1, 2) présentant respectivement un segment de prolongement en forme de bande (3, 4) qui entoure respectivement l'autre surface d'électrode (1, 2) au moins le long d'une partie de bordure partielle externe (15, 16) et est ainsi séparé par la zone isolante prolongée (8) de la surface d'électrode (1, 2) environnante respective,
les deux zones de bordure partielle externes (15, 16) s'étendant essentiellement à partir d'une extrémité de la zone de bordure partielle interne (9) autour de l'autre surface d'électrode respective (1, 2) au moins vers l'autre extrémité opposée de la zone de bordure partielle interne (9), et
les deux surfaces d'électrode de même taille (1, 2) étant conçues en demi-cercle au niveau de leur extrémité opposée à la pièce interne (9) de la zone isolée (8) en forme de lignes, ladite extrémité des segments de prolongement en forme de bande (3, 4) étant entourée en forme de faucille par les pièces externes (15, 16) de la zone isolante (8) en forme de lignes, respectivement.

2. Electrode plane selon la revendication 1, **caractérisée en ce qu'**une pièce interne (9) de la zone isolante (8) en forme de lignes et les zones de bordure partielle interne adjacentes des deux surfaces d'électrodes de même taille (1, 2) évoluent de façon linéaire et s'étendent le long d'un axe secondaire (6) de l'électrode plane.

3. Electrode plane selon l'une des revendications 1 ou 2, **caractérisée en ce que** les deux surfaces d'électrodes (1, 2) sont conçues de façon doublement symétrique autour d'un axe perpendiculairement au plan de l'électrode plane.

4. Electrode plane selon l'une des revendications précédentes 1 à 3, **caractérisée en ce que** des axes de raccordement (10, 11) sont disposés à une extrémité de l'un des segments de prolongement (3, 4) des électrodes planes (1, 2) et dans une zone de transition entre l'autre des surfaces d'électrodes (1, 2) et de l'autre segment de prolongement (3, 4).

5. Electrode plane selon la revendication 4, **caractérisée en ce que** les axes de raccordement (10, 11) s'étendent d'un côté de l'électrode plane dans le prolongement prévu de la pièce interne (9) le long de l'axe secondaire (6).

6. Electrode plane selon l'une des revendications précédentes, **caractérisée en ce que** les segments de prolongement (3, 4) s'éloignent respectivement dans une zone d'angle (13, 14) située le long de l'axe secondaire (6) des électrodes planes (1, 2), les zones d'angle (13, 14) étant situées à intervalles identiques par rapport à un axe principal (7) de l'électrode plane, et **en ce que** les segments de prolongement (3, 4) s'étendent respectivement autour des deux surfaces d'électrodes (1, 2) jusqu'à essentiellement à proximité de la zone d'angle (13, 14) de l'autre surface d'électrode.

7. Electrode plane selon l'une des revendications précédentes, **caractérisée en ce qu'**une proéminence (20, 21) des segments de prolongement en forme de bande (3, 4) s'étend respectivement le long de l'axe principal (7), dans l'autre surface d'électrode respective (1, 2).

8. Electrode plane, en particulier selon l'une des revendications 1 à 3, **caractérisée en ce que** des pattes de raccordement (24, 25) sont prévues dans la zone médiane, qui sont reliées aux surfaces d'électrodes (1, 2).

9. Electrode plane selon la revendication 1, **caractérisée en ce que** les segments de prolongement (3, 4) conduits autour de l'autre surface d'électrode respective (1, 2) sont acheminés ensuite à la zone de la surface d'électrode correspondante (1, 2) et y sont séparés par la zone isolante en forme de ligne (8) qui entoure d'autres segments de prolongement respectifs (3, 4).

10. Electrode plane selon la revendication 1, **caractérisée en ce que** les segments de prolongement en forme de bande (3, 4) sont conduits autour des deux surfaces d'électrodes (1, 2) en évoluant en forme de spirale l'un dans l'autre, deux fois ou plus.

11. Electrode plane selon l'une des revendications précédentes, **caractérisée en ce que** le long du parcours des segments de prolongement sous forme de bande (3, 4), celle-ci est commutée entre un ou plusieurs sites, de façon ininterrompue, et d'impédances (50, 51, 52, 53) pour former un gradient d'impédance.

12. Electrode plane selon la revendication 11, **caractérisée en ce que** l'axe secondaire (6) passe par les sites d'apposition des impédances (50, 51, 52, 53).
